# EUROPEAN PATENT APPLICATION

(11) **EP 4 559 393 A1**
(43) Date of publication of application: **28.05.2025**
(21) Application number: 23842499.8
(22) Date of filing: 21.07.2023
(51) Int. Cl.: A61B 5/1486, A61B 5/1482

(54) **DEVICE FOR MONITORING INTERSTITIAL LACTATE AND PH LEVELS**

(30) Priority: 22.07.2022 ES 202230679
(71) Applicant: Universidad de Cádiz, 11009 Cádiz (ES); Servicio Andaluz de Salud, 41071 Sevilla (ES); Ubrisecurity, S.L., 11600 Ubrique (Cádiz) (ES)
(72) Inventor: FERNÁNDEZ ALBA, Juan Jesús, 11510 Puerto Real (Cádiz) (ES); PALACIOS SANTANDER, José María, 11519 Puerto Real (Cádiz) (ES); CANO LABRADOR, José Manuel, 11600 Ubrique (Cádiz) (ES); CUBILLANA AGUILERA, Laura, 11519 Cádiz (ES); GARRIDO DOMÍNGUEZ, Pablo, 11600 Ubrique (Cádiz) (ES); GAMONOSO DE SAA, Rafael, 11600 Ubrique (Cádiz) (ES); GARCÍA GUZMAN, Juan José, 11009 Cádiz (ES); JIMÉNEZ HERAS, José Manuel, 11009 Cádiz (ES); GONZÁLEZ MACÍAS, Carmen, 11510 Puerto Real (Cádiz) (ES); RUIZ RODRÍGUEZ, Félix Alejandro, 11003 Cádiz (ES); VILAR SÁNCHEZ, Ángel, 11510 Puerto Real (Cádiz) (ES); QUINTERO PRADO, Rocío, 11510 Puerto Real (Cádiz) (ES); COBACHO DE ALBA, Juan Jesús, 11510 Puerto Real (Cádiz) (ES); SANTOTORIBIO CAMACHO, José Diego, 11510 Puerto Real (Cádiz) (ES)
(74) Representative: San Martin Alarcia, Esther
(86) International application number: PCT/ES2023/070477
(87) International publication number: WO 2024/018113

(57) **Abstract**

The invention relates to a microinvasive device for remotely and continuously monitoring interstitial lactate and pH levels in real time. The device comprises a body patch that adheres to the skin, said patch provided with one or more microneedles that extract interstitial body fluid. The patch incorporates a lactate amperometric electrochemical biosensor and a pH sensor, having the necessary electronics to acquire, adapt and transmit the signal that incorporates the values of the analytes recorded by the sensors to a wireless device with data processing capacity, where the doctor can view them.

## Description

### TECHNICAL FIELD

The present invention relates to the field of medicine and engineering, specifically to medical monitoring devices. It describes a device for continuous and real-time monitoring of various clinical parameters that are particularly relevant for determining metabolic acidosis in subjects undergoing intense physical exertion and, specifically, fetuses during labour and delivery, patients at risk of acidosis, or individuals engaged in sports activities.

### BACKGROUND OF THE INVENTION

Lactate, the ionized form of lactic acid, is the final product of anaerobic glucose metabolism in muscles. In mammals, L-lactate is the predominant enantiomer.

At rest, blood L-lactate concentration ranges between 0.5 and 2.0 mM [Chan, D., Barsan, M.M., Korpan, Y, Brett, C.M.A. (2017). L-lactate selective impedimetric bienzymatic biosensor based on lactate dehydrogenase and pyruvate oxidase. Electrochim Acta, 231:209-15, https://doi.org/10.1016/j.electacta.2017.02.050] and can increase to 8-10 mM during exercise, reaching 15-25 mM under intense physical exertion [Goodwin, M. L., Harris, J. E., Hernández, A., & Gladden, L. B. (2007). Blood Lactate Measurements and Analysis during Exercise: A Guide for Clinicians. J. Diabetes Sci. Technol., 1(4):558-569, https://doi.org/10.1177/193229680700100414]. As lactate concentration increases, cellular pH decreases due to proton accumulation within cells, potentially leading to blood acidosis if levels exceed 25 mM. When this lactate threshold is reached, various conditions may arise, ranging from mild fatigue to severe muscle pain.

Lactate biosensors have advanced significantly, particularly in sports medicine, for monitoring athlete homeostasis and evaluating endurance levels.

For example, WO2017173462 document describes a wearable sensor that adheres to the skin, enabling real-time lactate monitoring via electrochemical sensors detecting lactate in sweat. However, such devices cannot be used in other critical applications where lactate monitoring is essential.

Beyond exercise, any physiological condition that activates anaerobic metabolic pathways and disrupts lactate balance can lead to lactic acidosis. Conditions such as haemorrhagic shock, respiratory failure, renal failure, or tissue hypoxia may increase lactate production. Consequently, elevated lactate levels serve as an early warning signal for medical intervention in hospitalized patients.

Research has shown that combining lactate and pH measurements provides a more accurate and reliable diagnosis for various pathologies [Booth, M.A., Gowers, S.A.N., Hersey, M., Samper, I.C., Park, S., Anikeeva, P., Hashemi, P., Stevens, M.M., Boutelle, M.G. (2021). Fiber-Based Electrochemical Biosensors for Monitoring pH and Transient Neurometabolic Lactate. Anal. Chem., 93(17):6646-6655, https://doi.org/10.1021/acs.analchem.0c05108]. Therefore, a continuous pH sensor is recommended alongside lactate monitoring.

However, devices like those described in the WO2017173462 document are not suitable for biomedical applications since they rely on sweat analysis, requiring either spontaneous perspiration or induced sweating, which can be harmful to the patient. Additionally, these kind of measures in patients, coming from sweat, can be only made in an intermittent way and not continuously. It must be considered that the so-obtained punctual values of lactate levels may not significantly reflect the patient's overall physiological state and are insufficient for clinical assessment.

Other devices measure these parameters in interstitial fluid using microneedles, allowing for continuous monitoring with less invasiveness and pain compared to blood sampling via traditional needles. An example of these devices is described in the WO2021232109 document, where high-density microneedle arrays (2000/cm²) are employed. However, this type of devices may damage the skin, causing ulcers or tissue injuries.

Similar to athletes under exertion, foetuses experience significant stress during labour and delivery, that is why fetal well-being monitoring is crucial. The objective of this practice is to detect hypoxia or acidosis in order to avoid complications linked to these states, such as hypoxic-ischemic encephalopathy, cerebral palsy, or stillbirth.

Respiratory acidosis caused by carbon dioxide accumulation (hypercapnia) is common during normal metabolism, thus although the concentration of H⁺ ions increase, this type of transient acidosis is not associated with negative outcomes. However, when the foetus receives insufficient oxygen during labour/delivery, it loses the ability to maintain normal metabolism, forcing a shift to anaerobic metabolism. As a result, lactic acid is produced and accumulates, leading to a decrease in pH. This type of acidosis, known as metabolic acidosis, can lead to asphyxia during labour/delivery if oxygen deprivation persists for an extended period, increasing the risk of postnatal neurological complications such as hypoxic-ischemic encephalopathy or long-term disabilities. Severe metabolic hypoxia with lactate accumulation is considered strong indicator of poor perinatal outcomes [Wiberg, N., & Källén, K. (2017). Fetal scalp blood lactate during second stage of labor: determination of reference values and impact of obstetrical interventions. J. Matern. Fetal Neonatal Med. 30(5):612-617, https://doi.org/10.1080/14767058.2016.1181167], leading to fetal distress.

The current clinical method used to monitor fetal well-being is cardiotocographic recording, which continuously registers fetal heart rate and the intensity of uterine contractions. However, the specificity of this technique for detecting neonatal hypoxic-ischemic syndrome (oxygen deprivation) is so low that it sometimes results in unnecessary emergency interventions (e.g., caesarean sections), posing risks to both the mother and the foetus, in addition to the associated healthcare costs.

For this reason, when cardiotocographic monitoring yields concerning results, a second diagnostic test must be performed to confirm potential fetal homeostasis alterations before making clinical decisions.

Currently, this secondary test involves obtaining a fetal scalp blood sample to measure blood lactate levels and pH, both of which are indicative of metabolic acidosis. When fetal lactate levels exceed 5.2 mmol/L, the recommended course of action is to expedite delivery by the fastest possible method-typically an emergency caesarean section due to fetal distress [Ramanah, R., Martin, A., Riethmuller, D., Maillet, R., Schaal, J.-P. (2005). Value of fetal scalp lactate sampling during labor: a comparative study with scalp pH. Gynécologie Obstétrique & Fertilité, 33:107-112, https://doi.org/10.1016/j.gyobfe.2005.01.004; Birgisdottir, B.T., Holzmann, M., Varli, I.H., Graner, S., Saltvedt, S., & Nordström, L. (2017). Reference values for Lactate Pro 2TM in fetal blood sampling during labor: a cross-sectional study. J. Perinat. Med. 45:321-325, https://doi.org/10.1515/jpm-2016-0027].

The technique for obtaining these blood samples (performed intermittently and not recommended for more than two collections) is invasive, complex, and risky for both the mother and the foetus. Consequently, it is not routinely performed in all hospitals. In this context, the final decision to proceed with an urgent intervention (caesarean section) due to suspected fetal distress is often based solely on cardiotocographic monitoring, despite its known low reliability.

Studies indicate that among all caesarean sections performed due to unsatisfactory cardiotocographic results, only 36% of newborns exhibit sequelae. The remaining 64% correspond to healthy newborns, meaning that these urgent interventions, along with their associated risks, could have been avoided [Gluhovschi, A., luriciuc, M., Anastasiu, D., Cimpeanu, L., & Nyiredi, A. (2012). A retrospective analysis over the emergency Cesarean section performed due to cardiotocographic modifications. Timisoara Med. J., 62(1-2):20-23]. This clearly demonstrates that cardiotocographic monitoring cannot be considered a definitive diagnostic test. In Spain, the magnitude of the problem affects approximately 25,000 cases per year. Considering that about 100,000 cesarean sections performed in 2016 (according to the National Statistics Institute, INE), 25% were conducted due to suspected intrapartum fetal distress.

The significant discrepancy between the number of caesarean sections performed based on cardiotocographic fetal distress indicators and the actual number of cases that develop neonatal hypoxic-ischemic encephalopathy highlights the high falsepositive rate of current techniques. This underscores the urgent need for an alternative diagnostic method to reduce the number of unnecessary emergency interventions.

Devices such as the one described in patent WO2020069572 enable fetal lactate and pH monitoring during labour using needles equipped with lactate oxidase sensors. These needles take measurements from fetal blood and tissues, and their embedded sensors detect various parameters, including lactate and pH levels. However, despite being less cumbersome than other devices, they still require connecting cables between the measuring device and the receiver, as well as anchoring mechanisms to ensure the device to the foetus. This makes the system highly inconvenient and complex. Furthermore, these devices use needles containing biosensors, which introduce a risk of contamination if the needle breaks. The materials and reagents contained within the sensors could leak into the fetal blood and tissues, potentially causing toxicity. Additionally, the use of solid needles, as in this case, results in pain upon insertion and potential tissue damage.

Currently, biosensors for lactate detection include both bienzymatic sensors with two enzymes [Pundir, C.S., Narwal, V., Batra, B. (2016). Determination of lactic acid with special emphasis on biosensing methods: A review. Biosens. Bioelectron., 86:777-790, https://doi.org/10.1016/j.bios.2016.07.076; Andersson, A., Chen, Q., Groop, L., Bülow, L., Xie, B. (2017). Continuous and simultaneous determination of venous blood metabolites. Talanta, 171:270-274, https://doi.org/10.1016/j.talanta.2017.05.010; Kotanen, C.N., Karunwi, O., Alam, F., Uyehara, C.F.T., Guiseppi-Elie, A. (2018). Fabrication and in vitro performance of a dual responsive lactate and glucose biosensor. Electrochim. Acta, 267:71-79, https://doi.org/10.1016/j.electacta.2018.02.042; Tsuruoka, N., Ishii, K., Matsunaga, T., Nagatomi, R., Haga, Y. (2016). Lactate and glucose measurement in subepidermal tissue using minimally invasive microperfusion needle. Biomed. Microdevices, 18(1):19, https://doi.org/10.1007/s10544-016-0049-z], and single-enzyme sensors [Cunha-Silva, H., Arcos-Martinez, M.J. (2018). Dual range lactate oxidase-based screen-printed amperometric biosensor for analysis of lactate in diversified samples. Talanta, 188:779-787, https://doi.org/10.1016/j.talanta.2018.06.054; Lamas-Ardisana, P.J., Loaiza, O.A., Añorga, L., Jubete, E., Borghei, M., Ruiz, V., Ochoteco, E., Cabañero, G., Grande, H.J. (2014). Disposable amperometric biosensor based on lactate oxidase immobilized on platinum nanoparticle-decorated carbon nanofiber and poly(diallyldimethylammonium chloride) films. Biosensors and Bioelectronics, 56, 345-351. [https://doi.org/10.1016/j.bios.2014.01.047]; Andrus, L.P., Unruh, R., Wisniewski, N.A., & McShane, M.J. (2015). Characterization of lactate sensors based on lactate oxidase and palladium benzoporphyrin immobilized in hydrogels. Biosensors, 5, 398-416. [https://doi.org/10.3390/bios5030398]; Poscia, A., Messeria, D., Moscone, D., Ricci, F., & Valgimiglia, F. (2005). A novel continuous subcutaneous lactate monitoring system. Biosens. Bioelectron., 20:2244-2250, https://doi.org/10.1016/j.bios.2004.10.031]. Some of these biosensors are capable of quantifying lactate in interstitial tissue using minimally invasive techniques [Rassaei, L., Olthuis, W., Tsujimura, S., Sudhölter, E.J.R., van den Berg, A. (2014). Lactate biosensors: current status and outlook. Anal. Bioanal. Chem., 406:123-137, https://doi.org/10.1007/s00216-013-7307-1; Weltin, A., Kieninger, J., Urban, G.A. (2016). Microfabricated, amperometric, enzyme-based biosensors for in vivo applications. Anal. Bioanal. Chem., 408(17):4503-4521, https://doi.org/10.1007/s00216-016-9420-4; Pundir, C.S., Narwal, V., Batra, B. (2016). Determination of lactic acid with special emphasis on biosensing methods: A review. Biosens. Bioelectron., 86:777-790, https://doi.org/10.1016/j.bios.2016.07.076].

However, none of these biosensors have been specifically applied to lactate detection in fetal interstitial tissues.

### EXPLANATION OF THE INVENTION

In the present invention, the measurement of lactate is proposed using an electrochemical biosensor, which is associated with a simple, fast, bloodless technique (as it does not involve incisions), reliable, and minimally invasive, allowing for the real-time and continuous evaluation of lactate levels with the simple placement of the device. The proposed device combines a biological recognition element, which would sensitively and specifically monitor lactate and pH levels, with an electrochemical transduction system, due to the sensitivity, simplicity, and rapid response of this type of transducer compared to other optical or thermal biosensors [Kucherenko, I.S., Topolnikova, YV., Soldatkin, O.O. (2019). Advances in the biosensors for lactate and pyruvate detection for medical applications: A review. TrAC - Trends Anal. Chem.110:160-72, https://doi.org/10.1016/j.trac.2018.11.004].

The described device is useful for monitoring lactate and pH levels and detecting possible metabolic acidosis, both in individuals engaged in physical activity and those at rest, such as patients who require these parameters to be monitored. In these cases, the device can adhere to the skin on any part of the body of the individuals being monitored.

The device is also useful for intrapartum fetal well-being monitoring. In this application, the device is preferably attached to the fetal scalp during labour.

Monitoring is carried out remotely (wireless), continuously (online), and in real-time through a mobile device such as a smartphone or tablet.

The biosensor provides continuous measurements and real-time trend changes, offering significantly more relevant information than single blood sample measurements, greatly improving lactate estimation compared to the intermitent and invasive methods currently used in clinical practice.

A device that can adequately monitor intrapartum fetal well-being is proposed, providing sufficient, continuous, and real-time information, facilitating decision-making and proper labour management in pregnant women at risk of fetal distress. The device is minimally invasive and has the capability to transmit the collected information to a remote receiver (smartphone, tablet, laptop, etc.), providing physicians with an objective, simple, and easy-to-use tool that delivers a more accurate and reliable diagnosis than currently employed systems.

A device for the remote monitoring of intrapartum fetal well-being is described, based on the continuous and real-time determination of different clinical parameters, specifically interstitial lactate and interstitial pH levels.

More specifically, the present invention consists of a microinvasive device for remote, continuous, and real-time monitoring of intrapartum fetal well-being, based on the use of a body patch that can adhere to the skin of the fetal head, equipped with one or more needles that allow for the extraction of interstitial body fluid, incorporating an amperometric electrochemical lactate biosensor and a potentiometric pH sensor, along with the necessary electronics for signal acquisition, conditioning, and transmission, which incorporates the values of the analytes recorded by the sensors to a wireless device with data processing capabilities, where they are stored temporarily or permanently and can be visualized by the physician.

This lactate biosensor preferably features a Prussian Blue redox mediator configuration on a carbon substrate, covered with the following layers: lactate oxidase enzyme and gold nanoparticles, chitosan, polyvinyl chloride (PVC), and Nafion.

The adhesive patch includes one or more microneedles that facilitate the extraction of interstitial fluid. During initial placement, a first perforation is made in the skin layer to allow the sensors to work.

In the present document, a microneedle is defined as a needle with a length of less than 1000 microns, while a needle is considered to have a length ranging from 1000 to 2000 microns.

The microneedles enable the extraction of interstitial fluid samples from the monitored subjects, which are then analysed by the biosensor. This design ensures that even in the event of microneedle breakage, the biosensor and the reactants and materials contained within it will never come into contact with the subject's tissues, significantly enhancing the safety of the device.

The adhesive patch may contain one or more microneedles, preferably around 100 microneedles, arranged in the centre of the patch. The adhesive material is preferably located at the edge of the patch. The use of adhesive ensures the placement of the device and its adhesion to the skin during physical activity or, in the case of foetuses, during childbirth, without using a high density of microneedles for fixing the patch. It also eliminates the need for other auxiliary devices that could be highly uncomfortable (such as clamps or cables).

The lactate biosensor incorporated in the invention consists of a redox mediator (Prussian Blue) on top of the carbon substrate, covered by the following layers: lactate oxidase enzyme and gold nanoparticles, chitosan, polyvinyl chloride (PVC), and Nafion. The pH sensor in the invention is a carbon electrode electrodeposited with polyaniline.

The configuration of the layers used in the optimized lactate biosensor allows the measurement of lactate concentration in an indirect manner, by monitoring the hydrogen peroxide species generated as a by-product in the enzymatic reaction of lactate with lactate oxidase. The first Prussian Blue layer enables the detection of hydrogen peroxide at much lower electrical potential than conventional Pt-based sensors, significantly reducing the influence of interferents.

Furthermore, the enzymatic layer provides the necessary selectivity to detect lactate exclusively, even in complex matrices such as physiological fluids.

Additionally, gold nanoparticles, obtained through green synthesis, are added on top of the previous layer to improve sensitivity by increasing the biosensor's specific surface area and facilitating a shift of the signal to less positive potentials due to their electrocatalytic effect.

Although literature describes biosensors that may seem similar at first glance, there is no precedent for a biosensor that integrates these different polymeric layers in the combined manner described in this document.

Specifically, the chitosan layer is placed as a coating layer over the enzyme layer to encapsulate it and enhance its functionality. Moreover, this biopolymer is highly biocompatible, providing an optimal environment for the necessary enzymatic reactions to take place.

On the other hand, the PVC and Nafion layers significantly extend the sensor's linear concentration range, either as a diffusion membrane in the first case, or as an anionic reticulum in the second one. Notably, the latter serves a dual role: its anionic nature repels negatively charged interfering species (e.g., ascorbic acid, uric acid, etc.) and also slows down lactate diffusion into the biosensor, preventing early enzymatic saturation.

Additionally, the chitosan and Nafion membranes have been deposited using the "spin coating" method, which has not been previously used for the development of lactate biosensors. This approach produces much more compact and significantly less porous membranes than those resulting from traditional drop-casting deposition. This contributes to the robustness and further improves the device's useful linear concentration range, which is essential for the proposed applications.

The lactate biosensor unit is equipped with four contacts, three of which are electrodes: working electrode, reference electrode, counter electrode, and electrical contact. Meanwhile, the pH sensor unit has three contacts: working electrode, reference electrode, and electrical contact. These electrodes can be manufactured using conductive material that is either printed or etched, such as printed carbon or a metal sheet that can be etched.

The device described in this invention also includes the necessary electronics to enable the acquisition, conditioning, and transmission of the analyte values recorded by the sensors to an external wireless receiver with data processing capabilities.

Among the electronic components incorporated in the device are connection pins, a digital-to-analog converter, an amplifier, an analog-to-digital converter, a microcontroller, a Bluetooth transceiver, and a power supply.

With these electronic components, it is possible to establish the necessary potential at each pin for the proper functioning of the electrochemical sensors, as well as to acquire, modulate, and transmit the signals obtained from the sensors through the pins connected to the microneedles.

To establish the necessary potential on the sensor pins, a 12-bit digital-to-analog converter is available, connected to the reference pins, that will be directly linked to the connection pins of the microneedles through the tracks of the printed circuit.

This stage also includes temperature sensors necessary for the calibration of measurements.

The signal conditioning stage consists of filtering and amplifying the signals from the different sensors:
- Filtering: Low-pass filters are implemented before the signal amplification stage.
- Amplification: Transimpedance amplifiers are incorporated to convert the current signal into a voltage signal, which is then directly connected to the analog-to-digital converter.

Subsequently, it is necessary to convert the analogic signal into a digital signal that can be processed by the microcontroller. To achieve this, an Analog-to-Digital Converter (ADC) with an SPI (Serial Peripheral Interface) output is integrated and linked to the microcontroller input.

The microcontroller, in addition to providing the necessary signals for controlling each of the previous stages, receives the sensor data from the analog-to-digital conversion stage.

For measure data transmission to the receiving device, as well as for receiving various sensor configuration commands, the device includes a low-energy Bluetooth transceiver.

Finally, to power all the components, the device incorporates a power supply.

This architecture forms an electronic schematic, designed on a printed circuit board (PCB), where electromechanical elements and the power supply itself are also assembled.

Regarding data visualization, the collected information will be displayed on a remote receiver (such as a smartphone, tablet, or laptop) through a dedicated software application installed on the device. This enables physicians to easily interpret the data progression and extract relevant conclusions from historical records if necessary. This application will be subject to future improvements through software patches or firmware updates.

In addition to the evident advantages of having a minimally invasive device capable of adequately monitoring fetal well-being during labour and delivery -continuously and in real time, thus providing sufficient information for informed decision-making and proper management of labour in high-risk pregnancies-, several additional benefits are outlined below:
- Reduction in false positives associated with current diagnostic tools, which are predominantly based on the interpretation of cardiotocographic recordings.
- Lower caesarean section rates, with a global reduction of approximately 5% and up to 25% for emergency cases (according to the Spanish National Statistics Institute, INE, 2016), thereby reducing the associated costs (estimated at approximately €44 million annually nationwide). Additionally, material and human resources for labour care are reduced by 10%, while simultaneously improving sensitivity and specificity in detecting intrapartum fetal hypoxia.
- Improved efficiency in healthcare services, enabling physicians to monitor these parameters in a simple and effective manner.
- Reduced malpractice claims related to childbirth assistance.
- Increased skin-to-skin contact immediately after birth, which is often not possible in caesarean sections but has proven benefits during the hours following delivery.

In summary, the intensive monitoring of fetal metabolism through continuous measurement of lactate and pH levels-enabled by the device subject to this invention-represents a paradigm shift in the intrapartum fetal well-being assessment.

Furthermore, the real-time identification of pH values significantly aids in the diagnosis of metabolic acidosis, among other conditions, thereby unequivocally enhancing the reliability of the device.

Another aspect of the invention pertains to the design and manufacturing process of a microinvasive amperometric electrochemical lactate biosensor, whose configuration has been optimized for its use in the monitoring device subject to this invention.

Additionally, the device may be applied to any situation requiring continuous monitoring of pH and lactate levels in a subject.

Some potential applications include:
- Any clinical scenario requiring continuous pH and lactate monitoring.
- As an alert generator in an external wireless receiver in conditions associated with lactic acidosis.
- For use in neonatal intensive care units (NICUs) to aid in the diagnosis of neonatal sepsis.
- For close monitoring of pH and lactate levels in adult patients with diabetic ketoacidosis or sepsis.
- For sports medicine applications, enabling real-time monitoring of lactate and pH levels during athletic training.

### BRIEF DESCRIPTION OF THE DRAWINGS

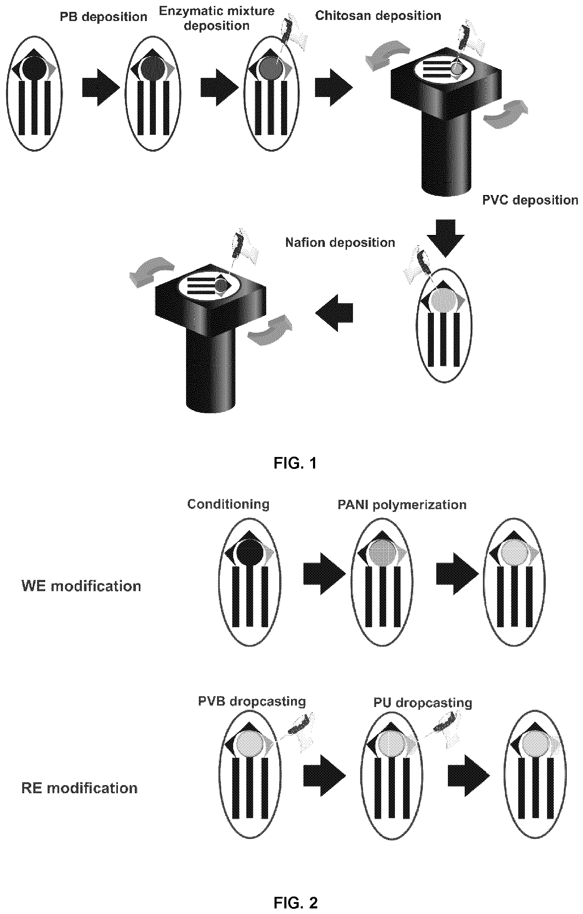

**Figure 1****.** Manufacturing process of the lactate biosensor.
**Figure 2****.** Manufacturing process of the pH sensor.
**Figure 3****.** Schematic representation of the chemical reaction occurring during lactate determination by the developed biosensor.
**Figure 4****.** Diagram of the electronic system architecture that constitutes the measurement system, composed of different functional blocks. This figure distinguishes the following components:
   - Connection pins (PC).
   - Digital-to-Analog Converter (DAC).
   - Amplifier (A).
   - Analog-to-Digital Converter (ADC).
   - Microcontroller (uC).
   - Bluetooth antenna (BLE).
   - Power supplier (PS).

### PREFERRED EMBODIMENT OF THE INVENTION

Below there are examples of embodiments for the components comprising the device subject to this invention:

### Example 1: Manufacturing of the lactate biosensor

### a) Materials and Reagents:

The following reagents were used for the biosensor fabrication:
- Carbon transducer.
- Solution of FeCl₃ and K₃Fe(CN)₆ (Merck-Sigma Aldrich).
- Lactate oxidase (Merck-Sigma Aldrich).
- Chitosan (Merck-Sigma Aldrich).
- Polyvinyl chloride (Merck-Sigma Aldrich).
- Colloidal solution of gold nanoparticles obtained following the procedure described in patent ES-2364914.

### b) Manufacturing Process of the Lactate Biosensor:

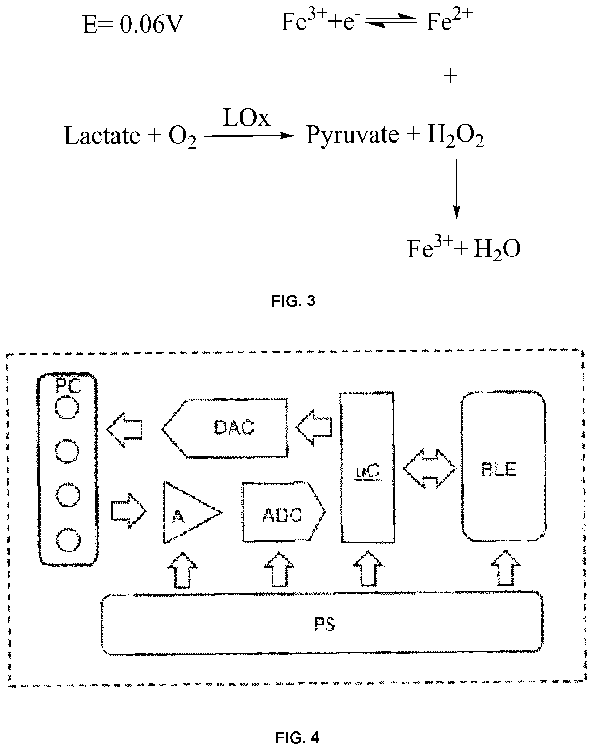

The manufacturing process of the lactate biosensor comprises the following steps:
1. A carbon transducer is used as the working electrode. This transducer undergoes pre-polarization in a phosphate buffer solution (concentration range: 0.01-0.1 M, pH range: 7-7.6, potential: 1.6-1.8 V) for approximately 200 seconds. Then, the following layers are sequentially deposited:
   a) Deposition of two droplets of a volume comprised between 10-20 µL each of FeCl₃ (0.1 M) / HCl (0.01 M) / KCI (0.1 M) and K₃Fe(CN)₆ / HCl (0.01 M) / KCI (0.1 M), respectively. The mixture is left to react for approximately 20 minutes, after which the excess volume is removed. Later, the electrode is washed with HCl (0.01 M) and then placed in an oven at a temperature comprised between 90-120°C for approximately one hour.
   b) Deposition of a 15-25 µL droplet of a solution containing 260 µL of Lactate Oxidase (6-10 enzymatic units) and gold nanoparticles (≈1.5 mM). This solution is dried under vacuum at -200 mbar for 2-3 hours.
   c) Deposition of a 20-40 µL droplet of 1% (w/v) chitosan in 0.1 M acetic acid (pH 4.25-4.75). The droplet is dried using a rotary system at a minimum speed of 1000 rpm.
   d) Deposition of a 4-5 µL droplet of 1% (w/v) polyvinyl chloride in tetrahydrofuran (99.5%). This droplet is dried at room temperature. This process is repeated from one to three times to form additional polymer layers.
   e) Deposition of a 10-13 µL droplet of 1% (w/v) Nafion in ethanol, with pH neutralized using ammonia (pH 7-8). The droplet is dried using a rotary system at a minimum speed of 1000 rpm.
2. The measurement is performed by applying an electric current to generate a potential (E) in the range of 0.00 to +0.06 V, where Fe³⁺ in the Prussian Blue surface is reduced to Fe²⁺. The Fe²⁺ then reacts with the hydrogen peroxide produced in the enzymatic reaction that converts lactate into pyruvate. The hydrogen peroxide, subsequently, re-oxidizes Fe²⁺ to Fe³⁺, generating a new electron flow that once again reduces the iron on the surface. The schematic representation of this reaction is shown in Figure 3. Therefore, the resulting current can be correlated with the hydrogen peroxide generated and, consequently, with the native lactate present in the sample.

### Example 2: Manufacturing of the pH sensor

### a) Materials and Reagents:

The following reagents were used for the biosensor fabrication:
- Carbon transducer.
- Hydrochloric acid (HCl) and aniline solution (Merck-Sigma Aldrich).
- Polyvinyl butyral (PVB) (Merck-Sigma Aldrich).
- Polyurethane (PU) (Merck-Sigma Aldrich).

### b) Manufacturing Process of the pH sensor:

1. A carbon transducer is used (previously preconditioned by cyclic voltammetry, with 10 sweeps from -0.3 to 1.1 V in a 0.5 M HCl solution. Later, the polyaniline layer is deposited as follows:
   - 12 cyclic voltammetry sweeps are performed in a potential range from -0.2 to 1.0 V in a solution containing 1 M HCl and 0.1 M aniline.
   To improve the stability of the pH measurement, the reference electrode is modified with additional layers of polyvinyl butyral (PVB) and polyurethane (PU) through:
   a) Deposition of a 2.5 µL droplet of a PVB solution (74.8 mg PVB, 50 mg NaCl in 1 mL methanol). This layer is left to air-dry for 1.5 hours and the process is repeated twice more. After finishing, the entire assembly is left to dry for 12 hours.
   b) Deposition of a 2.5 µL droplet of a PU solution (30 mg PU in 1 mL tetrahydrofuran, THF). This layer is air-dried for 24 hours.
2. The measurement is carried out by recording the potential difference between the working electrode and the reference electrode over time. The principle is based on the spontaneous phase modification of polyaniline according to the pH of the solution, with which is in contact. This phase change is associated with a change in the electrical potential, which can be monitored.

### Example 3: Fabrication of a Printed Circuit Board (PCB) Incorporating Electronic Components for Signal Acquisition, Processing, and Transmission

### a) Materials:

The following components were used in the fabrication of the printed circuit board:
- 32-bit dual-core microcontroller.
- System power supply based on one or more 3.3V low-dropout (LDO) voltage regulators.
- Bluetooth Low Energy (BLE) system.
- Sensor input adaptation system, composed of semiconductor elements and passive components.
- Four-layer PCB, designed to be immune to electromagnetic fields.

### b) Printed Circuit Board Manufacturing Process

The primary function of this module is to acquire signals from the sensors via pins connected to microneedles. It is also responsible for establishing the required potential at each pin to ensure the correct operation of the electrochemical sensors. The first stage of signal conditioning consists of filtering signals from different sensors. To achieve this, low-pass filters are used before feeding the signal into the amplification stage. Signal amplification is achieved through transimpedance amplifiers, allowing the signal to be read by the next module. The transimpedance amplifier converts the current signal into a voltage signal, which is then directly connected to the Analog-to-Digital Converter (ADC).

To set the required potential at the sensor pins, a 12-bit Digital-to-Analog Converter (DAC) is used, which is connected to reference pins that are directly linked, via PCB tracks, to the microneedle connection pins.

This stage also incorporates temperature sensors, which are necessary for measurement calibration.

Analog-to-Digital Converter (ADC): A 16-bit Sigma-Delta ADC is included to convert the analogic signal into a digital format, so it can be processed by the microcontroller. This ADC features an SPI (Serial Peripheral Interface) output, which connects directly to the microcontroller input.

Microcontroller / Bluetooth Low Energy Transceiver: The microcontroller provides the control signals required for each of the previous stages and receives the digitized sensor readings from the ADC stage. This stage incorporates a Bluetooth Low Energy (BLE) transceiver for the transmission of measurement data to the receiving device and for the reception of the different commands of the sensors configuration.

Power Supply Module: The power supply module provides energy to the entire system. It consists of a 3V CR2032 battery, as well as the low-dropout (LDO) voltage regulators and the filters and components required for proper voltage regulation and ripple reduction across the different power supply rails.

This architecture forms an electronic layout designed on a printed circuit board (PCB) that also integrates the electromechanical components and the battery.

All components are housed within an IP68-rated enclosure and will be fixed to the foetus scalp using a neutral adhesive dressing.

It should be understood that this description is not intended to be limited to the specific embodiments described, as variations may occur. Likewise, it should be noted that the terminology used is intended only to describe particular implementations and is not intended to be restrictive.

## Claims

1. Microinvasive device for remote, continuous, and real-time monitoring of interstitial lactate and pH levels, based on the use of a body patch that can adhere to the subject's skin, equipped with one or more microneedles for extracting interstitial body fluid, comprising:
- a lactate biosensor,
- a pH sensor, and
- circuits and electronic components for acquiring, conditioning, and transmitting the sensor signals to a wireless device with data processing capability.

2. Microinvasive device according to claim 1, suitable for fetal well-being monitoring during labour and delivery or in neonates.

3. Microinvasive device according to claim 1, suitable for its use in subjects during sporting activities.

4. Microinvasive device according to claim 1, suitable for its use in patients susceptible to metabolic acidosis.

5. Microinvasive device according to any of the preceding claims, where:
- the lactate biosensor consists of a redox mediator (Prussian Blue) on a carbon substrate covered with the following layers: lactate oxidase enzyme, gold nanoparticles, chitosan, polyvinyl chloride (PVC), and Nafion, and
- the pH sensor consists of a carbon electrode electrodeposited with polyaniline,
- circuits and electronic components for acquiring, conditioning, and transmitting the sensor signals to a wireless device with data processing capability.

6. Microinvasive device according to any of the preceding claims, which additionally incorporates the necessary electronics for acquiring, conditioning, and transmitting the signal containing the analyte values recorded by the sensors to a wireless device with data processing capability, where they are stored either temporarily or permanently, allowing the healthcare professional to visualize them.

7. Amperometric electrochemical lactate biosensor, optimized for its use in the microinvasive device according to any of the preceding claims, consisting of a redox mediator (Prussian Blue) on a carbon substrate covered with the following layers: lactate oxidase enzyme, gold nanoparticles, chitosan, polyvinyl chloride (PVC), and Nafion.

8. Method for manufacturing the lactate biosensor, according to claim 7, **characterized by** the following steps, starting from a pre-polarized carbon transducer in a phosphate buffer solution with a concentration in the range of 0.01-0.1 M, at a pH between 7 and 7.6, and at a potential between 1.6-1.8 V for approximately 200 s, acting as the working electrode:
a) Depositing two drops of volume 10-20 µL each of FeCl₃ (0.1 M) / HCl (0.01 M) / KCI (0.1 M) and K₃Fe(CN)₆ / HCl (0.01 M) / KCI (0.1 M), respectively, leaving a reaction time of approximately 20 minutes, then removing the volume and washing the electrode with HCl (0.01 M), followed by heating in an oven at 90-120°C for approximately one hour,
b) Depositing a drop of 15-25 µL of a solution composed of 260 µL of lactate oxidase (6-10 enzymatic units) and approximately 1.5 mM gold nanoparticles, then drying under vacuum at -200 mbar for 2-3 hours,
c) Depositing a drop of 20-40 µL of 1% (w/v) chitosan in acetic acid (0.1 M, pH 4.25-4.75 aprox.), then drying using a rotating rotor at a minimum of 1000 rpm,
d) Depositing a drop of 4-5 µL of 1% (w/v) polyvinyl chloride (PVC) in tetrahydrofuran (99.5%), then drying at room temperature, repeating this step one to three times to obtain additional polymeric layers,
e) Depositing a drop of 10-13 µL of 1% (w/v) Nafion in ethanol with pH neutralized with ammonia (pH 7-8 aprox.), then drying using a rotating rotor at a minimum of 1000 rpm.

9. Method for manufacturing the lactate biosensor, according to claim 7, **characterized by** the following steps, starting from a pre-polarized carbon transducer in a 0.1 M phosphate buffer solution at pH 7.4, at a potential of 1.7 V for 180 s, acting as the working electrode:
a) Depositing two drops of 12 µL each of FeCl₃ (0.1 M) / HCl (0.01 M) / KCI (0.1 M) and K₃Fe(CN)₆ / HCl (0.01 M) / KCI (0.1 M), respectively, leaving a reaction time of 20 minutes, then removing the volume and washing the electrode with 40 µL of HCl (0.01 M), followed by heating in an oven at 100°C for one hour,
b) Depositing a drop of 20 µL of a solution composed of 260 µL of lactate oxidase (7.7 enzymatic units) and approximately 1.5 mM gold nanoparticles, then drying under vacuum at -200 mbar for 2-3 hours,
c) Depositing a drop of 30 µL of 1% (w/v) chitosan in acetic acid (0.1 M, pH 4.5 aprox.), then drying using a rotating rotor at 1000 rpm,
d) Depositing a drop of 4.2 µL of 1% (w/v) polyvinyl chloride (PVC) in tetrahydrofuran (99.5%), then drying at room temperature, repeating this step twice more to obtain an additional polymeric layer,
e) Depositing a drop of 11.5 µL of 1% (w/v) Nafion in ethanol with pH neutralized with ammonia (pH 7 aprox.), then drying using a rotating rotor at 1000 rpm.
